# EUROPÄISCHE PATENTANMELDUNG

(11) **EP 0 668 278 A1**
(43) Veröffentlichungstag der Anmeldung: **23.08.1995**
(21) Anmeldenummer: 95101992.6
(22) Anmeldetag: 14.02.1995
(51) Int. Cl.: C07D 471/04, A61K 31/435

(54) **Adhäsionsrezeptor-Antagonisten**

(30) Priorität: 19.02.1994 DE 4405378
(71) Anmelder: MERCK PATENT GmbH, D-64293 Darmstadt (DE)
(72) Erfinder: Juraszyk, Horst, Dr., D-64342 Seeheim (DE); Gante, Joachim, Prof. Dr., D-64291 Darmstadt (DE); Wurziger, Hanns, Dr., D-64291 Darmstadt (DE); Bernotat-Danielowski, Sabine, Dr., D-61231 Badnauheim (DE); Melzer, Guido, Dr., D-65719 Hofheim/Ts. (DE)

(57) **Zusammenfassung**

Verbindungen der Formel I 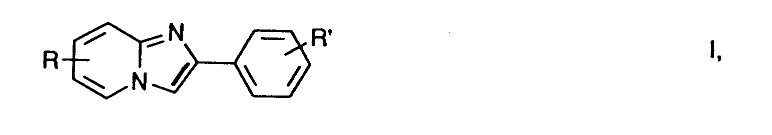
sowie die entsprechenden 5,6,7,8-Tetrahydroimidazo[1,2-a]pyridin-Derivate, deren physiologisch unbedenkliche Salze und/oder Solvate, hemmen die Bindung von Fibrinogen an den entsprechenden Rezeptor und können zur Behandlung von Thrombosen, Osteoporose, Tumorerkrankungen, Apoplexie, Herzinfarkt, Entzündungen, Arteriosklerose und osteolytischen Erkrankungen eingesetzt werden.

## Beschreibung

Die Erfindung betrifft Verbindungen der Formel I 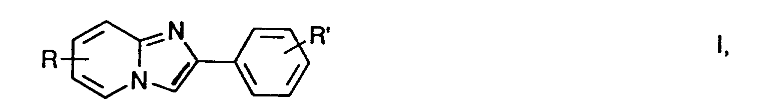
sowie die entsprechenden 5,6,7,8-Tetrahydro-imidazo[1,2-a]pyridin-Derivate,
worin
R Q oder COX,
R' Q, sofern R = COX und COX, sofern R = Q ist,
Q N0₂, NH₂, CN, CSNH₂, C(=NH)S-A, C(=NH)NHOH, C(=NH)-NH₂, CH₂-NH₂, CH₂NH-C(=NH)-NH₂, NH-C(=NH)-NH₂, CH₂NHCO-alk-NH₂, CH₂NHCO-Ar-C(=NH)NH₂, CH₂NHCO-Ar-CH₂-NH₂ oder D,
D 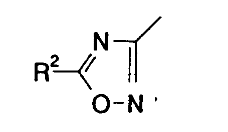
X OH, OA, AS, AS-AS', 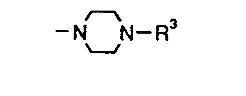
oder 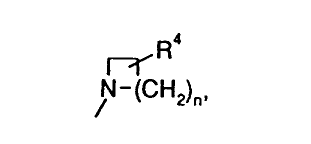
AS oder AS' jeweils unabhängig voneinander einen Aminosäurerest, ausgewählt aus einer Gruppe bestehend aus Ala, β-Ala, Arg, Asn, Asp, Gln, Glu, Gly, Leu, Lys, Orn, Phe, Pro, Sar, Ser, Thr, Tyr, Val, C-Allyl-Gly, C-Propargyl-Gly, N-Benzyl-Gly, N-Phenethyl-Gly, N-Benzyl-ß-Ala, N-Methyl-ß-Ala und N-Phenethyl-ß-Ala, wobei freie Amino- oder Carboxyl- gruppen auch mit an sich bekannten Schutzgruppen versehen sein können,
_{R}2 OH, A oder Ar,
R³ -(CH₂)ₘ-COOR⁵,
R⁴ -(CH₂)p-COOR⁵ oder -(CH₂)_{q}-O-(CH₂)ᵣ COOR⁵
_{R}5 H oder A,
m 1, 2 oder 3,
n 1,2, 3 oder 4,
p 0, 1 oder 2,
q 0 oder 1,
r 1 oder 2,
A Alkyl mit 1 bis 6 C-Atomen,
-alk- Alkylen mit 1 bis 6 C-Atomen

und
Ar Phenylen bedeuten,

sowie ihre physiologisch unbedenklichen Salze und/oder Solvate.

Verbindungen mit einem ähnlichen Wirkungsprofil, die jedoch eine andere Grundstruktur aufweisen, sind aus der EP-A1-0 381 033 bekannt.

Der Erfindung lag die Aufgabe zugrunde, neue Verbindungen mit wertvollen Eigenschafften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschatten besitzen. Insbesondere hemmen sie die Bindung von Fibrinogen, Fibronectin und des von Willebrand-Faktors an den Fibrinogenrezeptor der Blutplättchen (Glykoprotein Ilb/Illa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Entzündungen und Arteriosklerose verwendet werden. Ferner haben die Verbindungen einen Effekt auf Tumorzellen, indem sie deren Metastasierung hemmen. Somit können sie auch als Anti-Tumor-Mittel eingesetzt werden.

Desweiteren eignen sich die Verbindungen zur Prophylaxe und zur Behandlung von osteolytischen Erkrankungen, insbesondere von Osteoporose, Angiogenese und Restenose nach Angioplastie.

Die Verbindungen zeigen außerdem eine antimikrobielle Wirkung und können bei sämtlichen Behandlungen und Eingriffen eingesetzt werden, wo ein mikrobieller Befall verhindert werden muß.

Die Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen. Die Hemmung der Wechselwirkungen der β₃-Integrin-Rezeptoren mit geeigneten Liganden kann nach der Methode von J.W. Smith et al., J. Biol. Chem. 265, 12267-12271 (1990), nachgewiesen werden.

Gegenstand der Erfindung ist ferner ein Verfahren zur Herstellung einer Verbindung der angegebenen Formel I gemäß Anspruch 1 sowie von deren Salzen, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel 11 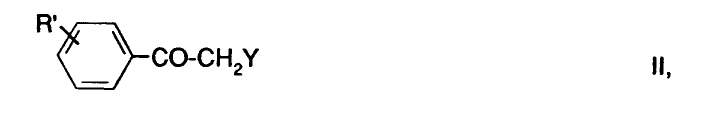
   worin
   R' die angegebene Bedeutung hat und
   Y Cl, Br, I, OH oder eine reaktionsfähige veresterte OH-Gruppe bzw. leicht nucleophil substituierbare Abgangsgruppe bedeutet,

   mit einem Pyridinderivat der Formel 111 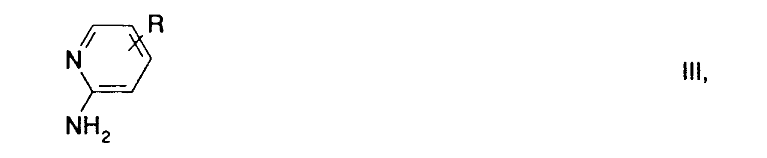
   worin R die angegebene Bedeutung besitzt, umsetzt, oder daß man
   (c) einen Rest R und/oder R' in einen anderen Rest R und/oder R' umwandelt, indem man
      - einen Ester der Formel I verseift, oder
      - eine Carbonsäure der Formel I verestert, in ein Amid überführt oder unter den Bedingungen der Peptidsynthese mit einer Aminosäure oder einem Dipeptid verknüpft, oder
      - eine N0₂- und/oder CN-Gruppe katalytisch hydriert, oder
      - eine Nitrilgruppierung durch Anlagerung von Ammoniak in eine C(=NH)-NH₂-Gruppe umwandelt, oder
      - eine Nitrilgruppe in eine Thiocarbamoylgruppe umwandelt, oder
      - eine Thiocarbamoylgruppe in eine Methylsulfimidogruppe überführt oder
      - eine Methylsulfimidogruppe in eine Amidin-Gruppe überführt
      - eine Nitrilgruppe durch Anlagerung von NH₂0H in eine C(=NH)-NHOH-Gruppe umwandelt, oder
      - eine C(=NH)-NHOH-Gruppe in eine Amidin-Gruppe umwandelt
      - eine CH₂NH₂-Gruppe in eine Alkanoylaminomethylgruppe umwandelt, oder
      - eine 1,2,4-Oxadiazol- bzw. 1,2,4-Oxadiazolinon-Gruppe in eine Amidin-Gruppe umwandelt, und/oder daß man
   (d) eine Verbindung der Formel I durch Behandeln mit einer Säure oder einer Base in eines ihrer Salze überführt.

Die vor- und nachstehend aufgeführten Abkürzungen von Aminosäureresten stehen für die Reste folgender Aminosäuren:
Ala Alanin
β-Ala ß-Alanin
Arg Arginin
Asn Asparagin
Asp Asparaginsäure
Asp (O But) Asparaginsäure-ß-butylester
Gln Glutamin
Glu Glutaminsäure
Gly Glycin
Leu Leucin
Lys Lysin
Orn Ornithin
Phe Phenylalanin
Pro Prolin
Sar Sarkosin (N-Methylglycin)
Ser Serin
Thr Threonin
Tyr Tyrosin
Val Valin.

Ferner bedeuten nachstehend:
BOC tert-Butoxycarbonyl
CBZ Benzyloxycarbonyl
DCCI Dicyclohexylcarbodiimid
DMF Dimethylformamid
EDCI N-Ethyl-N'-(3-dimethylaminopropyl)-carbodiimidhydrochlorid
Et Ethyl
Me Methyl
OMe Methylester
OEt Ethylester
TFA Trifluoressigsäure.

Vor- und nachstehend haben die Reste R und R' die bei der Formel I angegebene Bedeutung. Sofern eine Verbindung der Formel I ein chirales Zentrum besitzt, kann sie in mehreren enantiomeren Formen auftreten. Alle diese Formen und deren Gemische, insbesondere Racemate, werden von der Erfindung mit eingeschlossen.

In den vor- und nachstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, oder 3-Methylpentyl.

X ist vorzugsweise -OH, -OCH₃, -O-CH₂-CH₃, 4-Carboxypiperidino-, 4-Carboxymethoxypiperidino-, 4-Carboxymethylpiperazino-, 4-Carboxyethylpiperazino- oder steht besonders bevorzugt für einen Aminosäure- oder einen Dipeptidrest, der über eine Amidbindung an die Carbonylgruppe gebunden ist. Sofern X einen Aminosäure- oder Dipeptidrest bedeutet, sind die folgenden besonders bevorzugt: Ala, β-Ala, Gly, Arg sowie ß-Ala-Asp oder Sar.

Die Gruppe Q steht vorzugsweise für -NH₂, -C(=NH)-NH₂, -CH₂-NH₂, -CH₂-NH-CO-alk-NH₂, -CH₂-NH-CO-Ar-C(=NH)-NH₂, -CH₂-NH-CO-Alk-C(=NH)-NH₂, -CH₂-NH-CO-Ar-CH₂-NH₂, N0₂ oder CN. Ferner bedeutet Q außerdem bevorzugt -C(=NH)-S-A, -CSNH₂ oder -C(=NH)-NHOH.

Der Rest Ar steht für Phenylen, welches vorzugsweise über seine 1- und 4-Position an andere Reste gebunden ist.

Die Gruppe "-alk-" bedeutet vorzugsweise Alkylen mit 1 bis 5 C-Atomen, besonders bevorzugt -CH₂-, -(CH₂)₂-, -CH(CH₃)-, -(CH₂)₃-, -(CH₂)₄- oder -(CH₂)₅-.

Die Parameter m und n sind bevorzugt 1, femer aber auch 2 oder 3. Die Variable p ist vorzugsweise 0 oder 1, während q und r vorzugsweise 1 sind.

Unter den Verbindungen der Formel l sind diejenigen bevorzugt, in denen mindestens einer der angegebenen Reste, Gruppen und/oder Parameter eine der angegebenen bevorzugten Bedeutungen hat. Die Verbindungen der Formel I umfassen diejenigen der Formeln la und Ib, die der Formel I entsprechen, worin jedoch
in la R = Q und R' = COX bedeuten;
in Ib R = COX und R' = Q bedeuten;

Verbindungen der Formeln laa bis lan sind bevorzugt, die der Formel la entsprechen, worin jedoch zusätzlich
in laa Q = C(=NH)-NH₂ bedeutet;
in lab Q = C(=NH)-S-A bedeutet;
in lac Q = CSNH₂ bedeutet;
in lad Q = CN bedeutet;
in lae Q = CH₂-NH₂ bedeutet;
in laf Q = CH₂-NH₂, worin die NH₂-Gruppe durch eine Aminoschutzgruppe substituiert ist, bedeutet;
In lag Q = N0₂ oder NH₂ bedeutet;
in lah Q = CH₂-NH-CO-alk-NH₂, wobei "-alk-" geradkettig oder verzweigt sein kann, bedeutet;
in lai 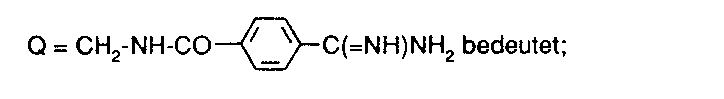
in laj 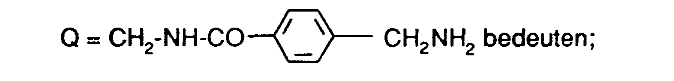
in lak X β-Ala oder β-Ala-Asp bedeutet, wobei die freie Säuregruppe des Aminosäurerestes gleichzeitig verestert sein kann;
in lal X = OH oder OA bedeutet;
in lam X einen in 4-Position substituierten Piperidin- oder Piperazinrest bedeutet;
in lan 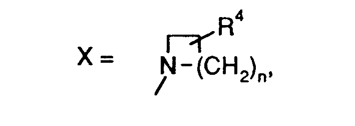
   bedeutet, wobei R⁴ für eine COOH-Gruppe steht.

Weiterhin sind Verbindungen der Formeln Iba bis Ibf bevorzugt, die den Formel Ib entsprechen, worin jedoch zusätzlich
in Iba Q = C(=NH)-NH₂ bedeutet;
in Ibb Q = C(= NH)-SA oder CSNH₂ bedeutet;
in Ibc Q = CN, NH₂, N0₂, C(=NH)-NHOH oder 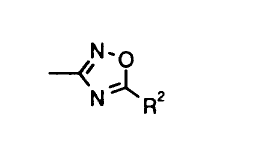 bedeutet;
in Ibd X Ala, β-Ala, ß-Ala-Arg oder einen der genannten C-terminal veresterten Aminosäure- oder Dipeptidreste bedeutet; in Ibe 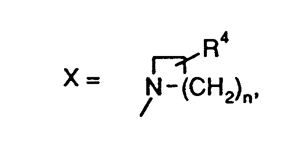 bedeutet, wobei R⁴ vorzugweise für COOH oder COOA steht und

in Ibf X = OH oder OA bedeutet.

Ferner sind bevorzugt Verbindungen, die an sich den Formeln laa bis lan bzw. Iba bis Ibf entsprechen, worin aber die Imidazopyridingruppe durch eine 5, 6, 7, 8-Tetrahydro-imidazo[1,2-a]pyridin-Gruppe ersetzt ist.

Desweiteren werden alle diejenigen Verbindungen von der Erfindung eingeschlossen, die über eine NH₂-Gruppe verfügen, wobei diese NH₂-Gruppe jedoch mit einer an sich bekannten Schutzgruppe versehen ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner J. March, Adv. Org. Chem., 3rd Ed. (1985), J. Wiley & Sons) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl. Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl. tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 500; vorzugsweise arbeitet man zwischen 15 und 30 °(Raumtemperatur).

Die BOC-Gruppe kann z.B. bevorzugt mit 40%iger Trifluoressigsäure in Dichlormethan oder mit etwa 3 bis 5 n HCI in Dioxan bei 15-60 °abgespalten weden, die FMOC-Gruppe mit einer etwa 5-20%igen Lösung von Dimethylamin, Diethylamin oder Piperidin in DMF bei 15-500. Eine Abspaltung der DNP-Gruppe gelingt z.B. auch mit einer etwa 3-10%igen Lösung von 2-Mercaptoethanol in DMF/Wasser bei 15-300.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100 und Drucken zwischen etwa 1 und 200 bar, bevorzugt bei 20-30 und 1-10 bar durchgeführt. Eine Hydrogenolyse der CBZ-Gruppe gelingt z.B. gut an 5-10%igem Pd-C in Methanol bei 20-300.

Ferner kann z.B. eine hydrogenolytische Umwandlung einer 1,2,4-Oxadiazolin-5-on-3-yl- bzw. einer 5-Alkyl-1,2,4-Oxadiazol-3-yl-Gruppe in eine Amidin-Gruppe durch katalytische Hydrierung vorgenommen werden.

Verbindungen der Formel I können auch bevorzugt durch Reaktion einer Verbindung der Formel II mit einem Pyridiederivat der Formel III erhalten werden. Dabei bedient man sich zweckmäßig der an sich bekannten Methoden der nucleophilen Substitution und/oder der N-Alkylierung von Aminen bzw. den Reaktionen zur Amidbildung.

Die Abgangsgruppe Y der Formel II bedeutet vorzugsweise Cl, Br, I, oder OH bzw. eine daraus ableitbare Gruppe wie z.B. die Trifluormethansulfonyloxy-, Toluolsulfonyloxy- oder Methansulfonyloxygruppe.

Die Reaktion gelingt vorzugsweise in Gegenwart einer zusätzlichen Base, z.B. eines Alkali- oder Erdalkalimetall-hydroxids oder -carbonats wie Natrium-, Kalium- oder Calciumhydroxid, Natrium-, Kalium-oder Cabiumcarbonat, in einem inerten Lösungsmittel z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 1200. Falls die Fluchtgruppe Y von I verschieden ist, empfiehlt sich ein Zusatz eines lodids wie Kaliumiodid.

Die Ausgangsstoffe der Formel 11 sind in der Regel bekannt oder können in Analogie zu bekannten Verbindungen hergestellt werden. Sie können z.B. hergestellt werden durch Reaktion eines substituierten Benzolderivates mit Säurechloriden der Formel CI-CO-CH₂-Y (Friedel-Crafts-Acylierung) in Gegenwart einer Lewis-Säure. Ferner können die Verbindungen der Formel 11 erhalten werden, indem man beispielsweise ausgehend von einem an der Methylgruppe entsprechend substituierten Acetophenon durch elektrophile Substitution den Phenylring modifiziert, oder daß man einen Rest R' in einen anderen Rest R' umwandelt, indem man beispielsweise eine Säuregruppe verestert bzw. mit einer Aminosäure oder einem Dipeptid verknüpft, oder indem man z.B. eine CN-Gruppe in eine CSNH₂-Gruppe umwandelt.

Ferner ist es möglich in einer Verbindung der Formel II einen Rest Y in einen anderen Rest Y umzuwandeln, z.B. dadurch, daß man eine OH-Gruppe (Y = OH) mit SOCI₂, SOBr₂, Methansulfonylchlorid oder p-Toluolsulfonylchlorid umsetzt.

Die Ryridinderivate der Formel III sind in der Regel bekannt und kommerziell erhältlich.

Die Umsetzung der Verbindungen der Formel II mit den Pyridinen der Formel III erfolgt in an sich bekannten Weise, bevorzugt in einem protischen oder aprotischen polaren inerten Lösungsmittel bei Temperaturen zwischen 20 und dem Siedepunkt des Lösemittels. Die Reaktionszeiten betragen 10 Min. bis 24 Std., vorzugsweise 2 Std. bis 10 Std.

Als Lösungsmittel eignen sich insbesondere auch Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander. Besonders geeignet ist N-Methylpyrrolidon.

Derivate mit freier primärer oder sekundärer Aminogruppe werden zweckmäßig in geschützter Form umgesetzt. Als Schutzgruppen kommen die zuvor genannten in Frage.

Weiterhin ist es möglich eine Verbindung der Formel I zu erhalten, indem man einen Rest R und/oder R' in einer Verbindung der Formel I in einen anderen Rest R und/oder R' umwandelt.

Insbesondere kann man Cyangruppen zu Aminomethylgruppen reduzieren oder in Amidinogruppen umwandeln, Carboxylgruppen verestern, Estergruppen spalten, Benzylgruppen hydrogenolytisch entfernen, Aminomethylgruppen in Guanidinomethylgruppen überführen.

Eine Reduktion von Cyangruppen zu Aminomethylgruppen gelingt zweckmäßigerweise durch katalytische Hydrierung, z.B. an Raney-Nickel bei Temperaturen zwischen 0 und 100°, vorzugsweise 10 und 300, und Drucken zwischen 1 und 200 bar, vorzugsweise bei Normaldruck, in einem inerten Lösungsmittel, z.B. einem niederen Alkohol wie Methanol oder Ethanol, zweckmäßig in Gegenwart von Ammoniak. Arbeitet man z.B. bei etwa 20 und 1 bar, so bleiben im Ausgangsmaterial vorhandene Benzylester- oder N-Benzylgruppen erhalten. Will man diese hydrogenolytisch spalten, so verwendet man zweckmäßig einen Edelmetallkatalysator, vorzugsweise Pd-Kohle, wobei man der Lösung eine Säure wie Essigsäure sowie auch Wasser zusetzen kann.

Zur Herstellung eines Amidins der Formel 1 kann man an ein Nitril der Formel I Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃1, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCI in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Analog sind die entsprechenden N-Hydroxy-amidine der Formel I aus den Nitrilen erhältlich, wenn man nach a) oder b), aber mit Hydroxylamin anstelle von Ammoniak arbeitet.

Zur Veresterung kann man eine Säure der Formel I mit einem Überschuß eines Alkohols behandeln, zweckmäßig in Gegenwart einer starken Säure wie Salzsäure oder Schwefelsäure bei Temperaturen zwischen 0 und 100, vorzugsweise 20 und 50 °.

Weiterhin kann man N-Hydroxy-amidine durch Umsetzung mit aliphatischen Carbonsäurechloriden in 1,2,4-Oxadiazole bzw. 1,2,4-Oxadiazolinone überführen und diese dann durch katalytische Hydrierung, z.B. an Raney-Ni, Pd/C oder Pt0₂, vorzugsweise in MeOH, Dioxan, Eisessig, Eisessig/Essigsäureanhydrid oder DMF, in Amidine überführen.

Umgekehrt kann ein Ester der Formel I, in die entsprechende Säure der Formel I umgewandelt werden, zweckmäßig durch Solvolyse nach einer der oben angegebenen Methoden, z.B. mit NaOH oder KOH in Wasser-Dioxan bei Temperaturen zwischen 0 und 400, vorzugsweise 10 und 300.

Eine Base der Formel 1 kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I durch Umsetzung mit entsprechenden Basen in ihre Metall- oder Ammoniumsalze umzuwandeln, z.B. ihre Natrium-, Kaum- oder Calciumsalze.

Die Verbindungen der Formel I, vornehmlich die Tetrahydroderivate, können ein oder mehrere chirale Zentren enthalten und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure.

Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/Acetonitril.

Natürlich ist es auch möglich, optisch-aktive Verbindungen der Formel 1 nach den oben beschriebenen Methoden zu erhalten, indem man Ausgangsstoffe (z.B. solche der Formel 11) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konseruierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in _{°}C angegeben. In den nachfolgenden Beispielen bedeutet "übliche Aufarbertung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. Sofern Molmassen angegeben werden, so werden herkömmliche massenspektroskopische Werte mit "M" und "fast atom bombardement"-Werte, sogenannte FAB-Werte, mit "M + 1 " bezeichnet.

### Beispiel 1

Eine Lösung von 0,54 g 3-[4-(7-Amidino-imidazo[1,2-a]pyridin-2-yl)-benzamido]-propionsäure-tert.-butylester [erhältlich nach Bsp. 2] in 6 ml Trifluoressigsäure wird 1 Std. bei Raumtemperatur gerührt. Die Lösung wird mit Diethylether versetzt und im Vakuum zur Trockne eingeengt. Das erhaltene Produkt wird mit Diethylether verrieben und abermals im Vakuum getrocknet. Man erhält 3-[4-(7-Amidino-imidazo[1,2-a]-pyridin-2-yl)-benzamido]-propionsäure-Bistrifluoracetat-Hydrat, F. 307°.

Analog erhält man durch Umsetzung mit Trifluoressigsäure ausgehend von 3-[4-(6-Amidino-imidazo[1,2-a]pyridin-2-yl)-benzamido]-propionsäure-tert.-butylester: 3-[4-(6-Amidno-imidazo[1,2-a]pyridin-2-yl)-benzamido]-propionsäure-Bistrifluoracetat.

### Beispiel 2

Eine Lösung von 1,8 g 3-[4-(7-Methylsulfimidoyl-imidazo[1,2-a]pyridin-2-yl)-benzamido]-propionsäure- tert.-butylester in Form des Hydroiodids [erhältlich nach Bsp. 4] werden in 150 ml Methanol gelöst und in Gegenwart von 1,5 g Ammoniumacetat 3 Tage bei Raumtemperatur gerührt. Nach Eindampfen erhält man durch übliche Aufarbeitung 3-[4-(7-Amidino-imidazo[1,2-a]pyridin-2-yl)-benzamido]-propionsäure-tert.-butylester, F. 172°.

Analog erhält man ausgehend von 3-[4-(6-Methylsulfimidoyl-imidazo[1,2-a]pyridin-2-yl)-benzamido]-propionsäure-tert.-butylester in Form des Hydroiodids: 3-[4-(6-Amidino-imidazo[1,2-a]pyridin-2-yl)-benzamido]-propionsäure-tert.-butylester; von 3-[4-(8-Methylsulfimidoyl-imidazo[1,2-a]pyridin-2-yl)benzamido]-propionsäure-tert.-butylester in Form des Hydroiodids: 3-[4-(8-Amidino-imidazo[1,2-a]pyridin-2-yl)-benzamido]-propionsäure-tert.-butylester.

### Beispiel 3

Man löst 1,5 g 3-[4-(7-Cyan-imidazo[1,2-a]pyridin-2-yl)-benzamido]-propionsäure-tert.-butylester [erhältlich nach Bsp. 5] in einem Lösungsmittelgemisch bestehend aus 22 ml Pyridin, 2,7 ml Triethylamin und 3 ml DMF und rührt 1,5 Std unter Eiskühlung, wobei während dieser Phase kontinuierlich H₂S-Gas eingeleitet wird. Anschließend rührt man die Reaktionsmischung noch 20 Std. bei Raumtemperatur. Nach Eindampfen und üblicher Aufarbeitung erhält man 3-[4-(7-Thiocarbamoyl-imidazo-[1,2-a]pyridin-2-yl)-benzamido]-propionsäure-tert.-butylester, F. 215 ° (Zers.).

### Beispiel 4

3-[4-(7-Thiocarbamoyl-imidazo [1,2-a]pyridin-2-yl)-benzamido]-propionsäure-tert.-butylester [erhältlich nach Bsp. 3] werden in 65 ml Aceton suspendiert und nach Zugabe von 3,3 ml Methyliodid 48 Std. bei Raumtemperatur gerührt. Der Niederschlag wird abgesaugt, mit 20 ml Aceton gewaschen und getrocknet. Man erhält 3-[4-(7-Methylsulfimidoyl-imidazo[1,2-a]pyridin-2-yl)-benzamido]-propionsäure-tert.-butylester-Hydroiodid, F. 193 °(Zers.).

### Beispiel 5

8,5 g 2-Amino-4-cyanpyridin sowie 27,5 g 4-Bromacetylbenzoesäure werden in 260 ml Ethanol 20 Std. gekocht. Nach Abkühlen wird der Niederschlag abgesaugt und wie üblich aufgearbeitet. Man erhält 4-(7-Cyanimidazo[1,2-a]pyridin-2-yl)-benzoesäure-Hydrobromid, F. >310 °.
Analog erhält man durch Umsetzung von 4-Bromacetylbenzoesäure mit 2-Amino-3-cyanpyridin:
4-(8-Cyanimidazo[1,2-a]pyridin-2-yl)-benzoesäure-Hydrobromid; mit 2-Amino-5-cyanpyridin:
4-(6-Cyanimidazo[1,2-a]pyridin-2yl)-benzoesäure-Hydrobromid.
Analog erhält man durch Umsetzung von 3-Bromacetylbenzoesäure mit 2-Amino-3-cyanpyridin:
3-(8-Cyanimidazo[1,2-a]pyridn-2-yl)-benzoesäure-Hydrobromid;

### Beispiel 6

6,6 g 4-(7-Cyanimidazo[1,2-a]pyridin-2-yl)-benzoesäure [erhältlich gemäß Bsp. 5] werden in 100 ml Dichlormethan und 25 ml DMF mit 4,5g β-Alanin-tert.-butylester in Gegenwart von 5,25 g N-(3-Dimethylaminopropyl)-N'-ethylcarbodiimid und 6 ml N-Methylmorpholin 20 Std. bei Raumtemperatur gerührt. Anschließend extrahiert man 3-4 mal mit je 30 ml Wasser, dampft ein und arbeitet wie üblich auf. Man erhält 3-[4-(7-Cyanimidazo[1,2-a]pyridin-2-yl)-benzamido]-propionsäure-tert.-butylester, F. 212 °(Zers.).

Analog erhält man durch Umsetzung:
von 4-(6-Cyanimidazo[1,2-a]pyridin-2-yl)-benzoesäure mit Piperidin-4-carbonsäure-tert.-butylester:
1-[4-(6-Cyanimidazo[1,2-a]pyridin-2-yl)-benzoyl]-piperidin-4-carbonsäure-tert.-butylester, F. 247 _{°} (Zers.). von 4-(6-Amidinoimidazo[1,2-a]pyridin-2-yl)-benzoesäure mit Piperidin-4-carbonsäure-tert.-butylester:
1-[4-(6-Amidinoimidazo[1,2-a]pyridin-2yl)-benzoyl]-piperidin-4-carbonsäure-tert.-butylester; von 4-(7-Amidinoimidazo[1,2-a]pyridin-2-yl)-benzoesäure mit Piperidin-4-carbonsäure-tert.-butylester:
1-[4-(7-Amidinoimidazo[1,2-a]pyridin-2-yl)-benzoyl]-piperidin-4-carbonsäure-tert.-butylester; von 4-(8-Amidinoimidazo[1,2-a]pyridin-2-yl)-benzoesäure mit Piperidin-4-carbonsäure-tert.-butylester:
1-[4-(8-Amidinoimidazo[1,2-a]pyridin-2-yl)-benzoyl]-piperidin-4-carbonsäure-tert.-butylester;

### Beispiel 7

1,4 g 1-[4-(6-Cyanimidazo[1,2-a]pyridin-2-yl)-benzoyl]-piperidin-4-carbonsäure-tert.-butylester [erhältlich nach Bsp. 6] werden in 140 ml Methanol in Gegenwart von 1,1 g Di-tert.-butyldicarbonat an Pd-Kohle (5%ig) hydriert. Anschließend wird der Katalysator abfiltriert, die Reaktionsmischung eingeengt und wie üblich aufgearbeitet. Man erhält nach Chromatographie über Kieselgel/Ethylacetat 1-[4-(6-tert.-Butoxycarbonylaminomethyl-imidazo[1,2-a]pyridin-2-yl)-benzoyl]-piperidin-4-carbonsäure-tert.-butylester, F. 158-162 °.

Zusätzlich erhält man bei der chromatographischen Trennung durch Eluieren mit Dichlormethan/Methanol als starker polare Komponente: 1-[4-(6-tert.-Butoxycarbonyl-aminomethyl-5,6,7,8-tetrahydroimidazo[1,2-a]pyridin-2-yl)-benzoyl]-piperidin-4-carbonsäure-tert.-butylester.

Analog erhält man ausgehend
von 4-(7-Cyanimidazo[1,2-a]pyridin-2-yl)-benzoesäure: 4-(7-tert.-Butoxycarbonyl-aminomethyl-imidazo[1,2-a]pyridin-2-yl)-benzoesäure-tert.-butylester und 4-(7-tert.-Butoxycarbonyl-aminomethyl-5,6,7,8-tetrahydro-imidazo[1,2-a]pyridin-2-yl)-benzoesäure-tert.-butylester;
von 4-(8-Cyanimidazo [1,2-a]pyridin-2-yl)-benzoesäure: 4-(8-tert.-Butoxycarbonyl-aminomethyl-imidazo[1,2-a]pyridin-2-yl)-benzoesäure-tert.-butylester und 4-(8-tert.-Butoxycarbonyl-aminomethyl-5,6,7,8-tetrahydroimidazo[1,2-a]pyridin-2-yl)-benzoesäure-tert.-butylester;
von 4-(6-Cyanimidazo[1,2-a]pyridin-2-yl)-benzoesäure: 4-(6-tert.-Butoxycarbonyl-aminomethyl-imidazo[1,2-a]pyridin-2-yl)-benzoesäure-tert.-butylester und 4-(6-tert.-Butoxycarbonyl-aminomethyl-5,6,7,8-tetrahydroimidazo[1,2-a]pyridin-2-yl)-benzoesäure-tert.-butylester;
von 3-(8-Cyanimidazo[1,2-a]pyridin-2-yl)-benzoesäure; 3-(8-tert.-Butoxycarbonyl-aminomethyl-imidazo[1,2-a]pyridin-2-yl)-benzoesäure-tert.-butylester und 3-(8-tert.-Butoxycarbonyl-aminomethyl-5,6,7,8-tetrahydro-imidazo[1,2-a]pyridin-2-yl]-benzoesäure-tert.-butylester.

### Beispiel 8

Man rührt eine Lösung von 0,64 g 1-[4-(6-tert.-Butoxycarbonyl-aminomethyl-imidazo[1,2-a]pyridin-2-yl)-benzoyl]-piperidin-4-carbonsäure-tert.-butylester [erhältlich gemäß Bsp. 7] in 7 ml Trifluoressigsäure 0,5 Std. bei Raumtemperatur. Anschließend fügt man 50 ml Toluol hinzu, entfernt das Lösungsmittel und verreibt den Rückstand mit Diethylether. Nach Trocknen erhält man 1-[4-(6-Aminomethyl-imidazo[1,2-a]-pyridin-2-yl)-benzoyl]-piperidin-4-carbonsäure-Bistrifluoracetat, F. 148_{°} (Zers.).

Analog erhält man durch Behandlung mit Trifluoressigsäure:

aus 1-[4-(7-tert.-Butoxycarbonyl-aminomethyl-imidazo[1,2-a]pyridin-2-yl)-benzoyl]-piperidin-4-carbonsäure- tert.-butylester:
1-[4-(7-Aminomethyl-imidazo[1,2-a]pyridin-2-yl)-benzoyl]-piperidin-4-carbonsäure-Bistrifluoracetat; aus 1-[4-(7-tert.-Butoxycarbonyl-aminomethyl-5,6,7,8-tetrahydro-imidazo[1,2-a]pyridin-2-yl)-benzoyl]-piperidin-4-carbonsäure-tert.-butylester;
1-[4-(7-Aminomethyl-5,6,7,8-tetrahydro-imidazo[1,2-a]pyridin-2-yl)-benzoyl]-piperidin-4-carbonsäure-Bistrifluoracetat;
aus 1-[4-(6-tert.-Butoxycarbonyl-aminomethyl-5,6,7,8-tetrahydro-imidazo[1,2-a]pyridin-2-yl)-benzoyl]-piperidin-4-carbonsäure-tert.-butylester:
1-[4-(6-Aminomethyl-5,6,7,8-tetrahydro-imidazo[1,2-a]pyridin-2-yl)-benzoyl]-piperidin-4-carbonsäure-Bistrifluoracetat;
aus 3-[4-(7-tert.-Butoxycarbonyl-aminomethyl-imidazo[1,2-a]pyridin-2-yl)-benzamido]-propionsäure-tert.-butylester:
3-[4-(7-Aminomethyl-imidazo[1,2-a]pyridin-2-yl)-benzamido]-propionsäure-Bistrifluoracetat;
aus 3-[4-(7-tert.-Butoxycarbonyl-aminomethyl-5,6,7,8-tetrahydro-imidazo[1,2-a]pyridin-2-yl)-benzamido]-pro- pionsäuretert.-butylester:
3-[4-(7-Aminomethyl-5,6,7,8-tetrahydro-imidazo[1,2-a]pyridin-2-yl)-benzamido]-propionsäure-Bistrifluoracetat, F. 183_{°} (Zers.);
von 4-(7-tert.-Butoxycarbonyl-aminomethyl-imidazo[1,2-a]pyridin-2-yl)-benzoesäure-tert.-butylester:
4-(7-Aminomethyl-imidazo[1,2-a]pyridin-2-yl)-benzoesäure-Bistrifluoracetat;
von 4-(7-tert.-Butoxycarbonyl-aminomethyl-5,6,7,8-tetrahydro-imidazo[1,2-a]pyridin-2-yl)-benzoesäure-tert.-butylester:
4-(7-Aminomethyl-5,6,7,8-tetrahydro-imidazo[1,2-a]pyridin-2-yl)-benzoesäure-Bistrifluoracetat;
von 4-(8-tert.-Butoxycarbonyl-aminomethyl-imidazo[1,2-a]pyridin-2-yl)-benzoesäure-tert.-butylester:
4-(8-Aminomethyl-imidazo[1,2-a]pyridin-2-yl)-benzoesäure-Bistrifluoracetat;
von 4-(8-tert.-Butoxycarbonyl-aminomethyl-5,6,7,8-tetrahydro-imidazo[1,2-a]pyridin-2-yl)-benzoesäure-tert.-butylester:
4-(8-Aminomethyl-5,6,7,8-tetrahydro-imidazo[1,2-a]pyridin-2-yl)-benzoesäure-Bistrifluoracetat;
von 4-(6-tert.-Butoxycarbonyl-aminomethyl-imidazo[1,2-a]pyridin-2-yl)-benzoesäure-tert.-butylester;
4-(6-Aminomethyl-imidazo[1,2-a]pyridin-2-yl)-benzoesäure-Bistrifluoracetat;
von 4-(6-tert.-Butoxycarbonyl-aminomethyl-5,6,7,8-tetrahydro-imidazo[1,2-a]pyridin-2-yl)-benzoesäure-tert.-butylester:
4-(6-Aminomethyl-5,6,7,8-tetrahydro-imidazo[1,2-a]pyridin-2-yl)-benzoesäure-Bistrifluoracetat;
von 3-(8-tert.-Butoxycarbonyl-aminomethyl-imidazo[1,2-a]pyridin-2-yl)-benzoesäure-tert.-butylester:
3-(8-Aminomethyl-imidazo[1,2-a]pyridin-2-yl)-benzoesäure-Bistrifluoracetat;
von 3-(8-tert.-Butoxycarbonyl-aminomethyl-5,6,7,8-tetrahydro-imidazo[1,2-a]pyridin-2-yl)-benzoesäure-tert.-butylester:
3-(8-Aminomethyl-5,6,7,8-tetrahydro-imidazo[1,2-a]pyridin-2-yl)-benzoesäure-Bistrifluoracetat.

### Beispiel 9

Analog Beispiel 7 erhält man ausgehend von 3-[4-(7-Cyanimidazo[1,2-a]pyridin-2-yl)-benzoyl]-piperidin-4-carbonsäure-tert.-butylester [erhältlich nach Bsp. 6] nach Umsetzung mit Di-tert.-butyldicarbonat und Hydrierung an Pd-Kohle (5%ig) 3-[4-(7-tert.-Butoxycarbonyl-aminomethyl-imidazo[1,2-a]pyridin-2-yl)-benzoyl]-piperidin-4-carhonsäure-tert.-butylester.

Als stärker polare Komponente erhält man zusätzlich 3-[4-(7-tert.-Butoxycarbonyl-aminomethyl-5,6,7,8-tetrahydro-imidazo[1,2-a]pyridin-2-yl)-benzoyl]-piperidin-4-carbonsäuretert.-butylester, M + 1: 498 (FAB).

### Beispiel 10

Analog Beispiel 5 erhält man ausgehend von 2,3-Diamino-pyridin durch Umsetzung mit 4-Bromacetylbenzoesäure 4-(8-Amino-imidazo[1,2-a]pyridin-2-yl)-benzoesäure-Hydrobromid, F. > 280 °(Zers.) (M: 253).

Analog erhält man:
aus 2,3-Diamino-pyridin durch Umsetzung mit 3-Bromacetylbenzoesäure 3-(8-Amino-imidazo[1,2-a]pyridin-2-yl)-benzoesäure-Hydrobromid, F > 300 °(Zers.) (M: 253).

### Beispiel 11

Man löst 2,2 g 4-(8-Amino-imidazo[1,2-a]pyridin-2-yl)-benzoesäure-Hydrobromid in 100 ml DMF und rührt nach Zugabe von einem Äquivalent tert.-Butyloxycarbonylazid 2 Std. bei Raumtemperatur. Nach üblicher Aufarbeitung erhält man 4-(8-tert.-Butoxycarbonyl-amino-imidazo[1,2-a]pyridin-2-yl)-benzoesäure-Hydrobromid.

Analog erhält man
aus 3-(8-Amino-imidazo[1,2-a]pyridin-2-yl)-benzoesäure-Hydrobromid: 3-(8-tert.-Butoxycarbonyl-amino- imidazo[1,2-a]pyridin-2-yl)-benzoesäure-Hydrobromid.

### Beispiel 12

Analog Beispiel 6 erhält man ausgehend von 4-(8-BOC-Amino-imidazo[1,2-a]pyridin-2-yl)-benzoesäure [erhältlich gemäß Bsp. 11] durch Umsetzung mit β-Alanin-tert.-butylester in Gegenwart von 5,25 g N-(3-Dimethyl-aminopropyl)-N'-ethylcarbodiimid und 6 ml N-Methylmorpholin bei Raumtemperatur nach üblicher Aufarbeitung: 3-[4-(8-BOC-Amino-imidazo[1,2-a]pyridin-2-yl)-benzamido]-propionsäure-tert.-butylester.

Analog erhält man durch Umsetzung mit β-Alanin-tert.-butylester ausgehend
von 3-(8-BOC-Amino-imidazo[1,2-a]pyridin-2-yl)-benzoesäure: 3-[3-(8-BOC-Amino-imidazo[1,2-a]pyridin-2-yl)-benzamido]-propionsäure-tert.-butylester.
Analog erhält man durch Umsetzung mit β-Ala-Asp(OBut)-tert.-butylester ausgehend von 3-(8-BOC-Amino-imidazo[1,2-a]pyridin-2-yl)-benzoesäure:
3-(8-BOC-Amino-imidazo[1,2-a]pyridin-2-yl)-benzoyl-β-Ala-Asp(OBut)-tert.-butylester; von 4-(8-BOC-Amino-imidazo[1,2-a]pyridin-2-yl)-benzoesäure:
4-(8-BOC-Amino-imidazo[1,2-a]pyridin-2-yl)-benzoyl-β-Ala-Asp(OBut)-tert.-butylester; von 3-(8-BOC-Amino-5,6,7,8-tetrahydro-imidazo[1,2-a]pyridin-2-yl)-benzoesäure:
3-(8-BOC-Amino-5,6,7,8-tetrahydro-imidazo[1,2-a]pyridin-2-yl)-benzoyl-ß-Ala-Asp(OBut)-tert.-butylester; von 4-(8-BOC-Amino-5,6,7,8-tetrahydro-imidazo[1,2-a]pyridin-2-yl)-benzoesäure:
4-(8-BOC-Amino-5,6,7,8-tetrahydro-imidazo[1,2-a]pyridin-2-yl)-benzoyl-β-Ala-Asp(OBut)-tert.-butylester.

### Beispiel 13

Analog Beispiel 8 erhält man nah Entfernung der Aminoschutzgruppen und Verseifung ausgehend von 3-[4-(8-BOC-Amino-imidazo[1,2-a]pyridin-2-yl)-benzamido]-propionsäure-tert.-butylester:
3-[4-(8-Amino-imidazo[1,2-a]pyridin-2-yl)-benzamido]-propionsäure-Bistrifluoracetat, F. 128-130 (Zers.);
von 3-[3-(8-BOC-Amino-imidazo[1,2-a]pyridin-2-yl)-benzamido]-propionsäure-tert.-butylester:
3-[3-(8-Amino-imidazo(1,2-a]pyridin-2-yl)-benzamido]-propionsäure-Bistrifluoracetat, F. 260-262 _{°} (Zers.);
von 3-(8-BOC-Amino-imidazo[1,2-a]pyridin-2-yl)-benzoyl-β-Ala-Asp(OBut)-tert.-butylester:
3-(8-Amino-imidazo[1,2-a]pyridin-2-yl)-benzoyl-ß-Ala-Asp-OH (Bistrifluoracetat), F. 308° (Zers.) (M: 439);
von 4-(8-BOC-Amino-imidazo(1,2-a]pyridin-2-yl)-benzoyl-β-Ala-Asp(OBut)-tert.-butylester
4-(8-Amino-imidazo[1,2-a]pyridin-2-yl)-benzoyl-ß-Ala-AspOH (Bistrifluoracetat), F. > 150 °(Zers.) (M + 1: 440);
von 3-(8-BOC-Amino-5,6,7,8-tetrahydro-imidazo[1,2-a]pyridin-2-yl)-benzoyl-β-Ala-Asp(OBut)-tert.-butylester: 3-(8-Amino-5,6,7,8-tetrahydro-imidazo[1,2-a]pyridin-2-yl)-benzoyl-β-Ala-Asp-OH (Bistrifluoracetat);
von 4-(8-BOC-Amino-5,6,7,8-tetrahydro-imidazo[1,2-a]pyridin-2-yl)-benzoyl-β-Ala-Asp(OBut)-tert.-butylester 4-(8-Amino-5,6,7,8-tetrahydroimidazo[1,2-a]pyridin-2-yl)-benzoyl-β-Ala-Asp-OH (Bistrifluoracetat).

### Beispiel 14

Analog Beispiel 1 erhält man ausgehend von den Estern aus Beispiel 6 durch Verseifung die folgenden Imidazo[1,2a]pyridin-Derivate:
1-[4-(6-Cyanimidazo[1,2-a]pyryridin-2-yl)-benzoyl]-piperidin-4-carbonsäure-Bistrifluoracetat;
1-[4-(6-Amidinoimidazo[1,2-a]pyridin-2-yl)-benzoyl]-piperidin-4-carbonsäure-Bistrifluoracetat;
1-[4-(7-Amidinoimidazo[1,2-a]pyridin-2-yl)-benzoyl]-pipendin-4-carbonsäure-Bistrifluoracetat;
1-[4-(8-Amidinoimidazo[1,2-a]pyridin-2-yl)-benzoyl]-piperidin-4-carbonsäure-Bistrifluoracetat.

### Beispiel 15

Man gibt zu einer Lösung von 2,1 g 4-(8-Aminomethyl-imidazo[1,2-a]pyridin-2-yl)-benzoesäure-tert.-butylester in 80 ml Dichlormethan ein Äquivalent BOC-Ala-OH und rührt 18 Std. bei Raumtemperatur in Gegenwart von 3,25 g N-(3-Dimethyl-aminopropyl)-N'-ethylcarbodiimid und 6 ml N-Methylmorpholin. Anschließend extrahiert man 3-4mal mit Wasser, dampft ein und arbeitet wie üblich auf. Man erhält 4-[8-(BOC-Ala-aminomethyl)-imidazo[1,2-a]pyridin-2-yl]-benzoesäure-tert.-butylester.

Analog erhält man durch Umsetzung von 4-(8-Aminomethyl-imidazo[1,2-a]pyridin-2-yl)-benzoesäure- tert.-butyl ester
mit 6-BOC-Aminohexansäure den 4-[8-(6-BOC-Aminohexanoyl-aminomethyl)-imidazo[1,2-a]pyridin-2-yl]-benzoesäure-tert.-butylester.
Analog erhält man durch Umsetzung von 3-(8-Aminomethyl-imidazo[1,2-a]pyridin-2-yl)-benzoesäure- tert.-butylester
mit 6-BOC-Aminohexansäure den 3-[8-(6-BOC-Aminohexanoyl-aminomethyl)-imidazo[1,2-a]pyridin-2-yl]-benzoesäure-tert.-butylester;
mit 3-BOC-Aminopropionsäure den 3-[8-(3-BOC-Aminopropionyl-aminomethyl)-imidazo[1,2-a]pyridin-2-yl]-benzoesäure-tert.-butylester.
Analog erhält man durch Umsetzung von 4-(8-Aminomethyl-5,6,7,8-tetrahydroimidazo[1,2-a]pyridin-2-yl)-benzoesäure-tert.-butylester
mit 6-BOC-Aminohexansäure den
4-[8-(6-BOC-Aminohexanoyl-aminomethyl)-5,6,7,8-tetrahydroimidazo[1,2-a]pyridin-2-yl]-benzoesäure- tert.-butylester;
mit 3-BOC-Aminopropionsäure den
4-[8-(3-BOC-Aminopropionyl-aminomethyl)-5,6,7,8-tetrahydro-imidazo[1,2-a]pyridin-2-yl]-benzoesäure- tert.-butylester;
mit 4-BOC-Aminomethylbenzoesäure den
4-[8-(4-BOC-Aminomethylbenzoyl-aminomethyl)-5,6,7,8-tetrahydro-imidazo[1,2-a]pyridin-2-yl]-benzoesaure-tert.-butylester.

### Beispiel 16

Analog Beispiel 15 erhält man durch Umsetzung von 4-(8-Aminomethyl-imidazo[1,2-a]pyridin-2-yl)-benzoesäureethylester mit 4-BOC-Amidinobenzoesäure den 4-[8-(4-BOC-Amidinobenzoyl-aminomethyl)-imidazo[1,2-a]pyridin--2-yl]-benzoesäureethylester; von 3-[3-(8-Aminomethyl-imidazo[1,2-a]pyridin-2-yl)-benzamido]-propionsäure mit 3-BOC-Aminopropionsäure die 3-[3-(8-(3-BOC-Aminopropionyl-aminomethyl)-imidazo[1,2-a]pyridin-2-yl)-benzamido]-propionsäure.

### Beispiel 17

Analog Beispiel 8 erhält man durch Entfernung der Aminoschutzgruppen und Verseifung der tert.-Butylester mit Trifluoressigsäure die folgenden Verbindungen in Form ihrer Trifluoracetate:
aus 4-[8-(BOC-Ala-Aminomethyl)-imidazo[1,2-a]pyridin-2-yl]-benzoesäure:
4-[8-(Ala-Aminomethyl)-imidazo[1,2-a]pyridin-2-yl]-benzoesäure, F. > 210 _{°} (Zers.) (M + 1: 339);
aus 4-[8-(6-BOC-Aminohexanoyl-aminomethyl)-imidazo[1,2-a]pyridin-2-yl]-benzoesäure:
4-[8-(6-Aminohexanoyl-aminomethyl)-imidazo[1,2-a]pyridin-2-yl]-benzoesäure, F. 234-240 °;
aus 3-[8-(6-BOC-Aminohexanoyl-aminomethyl)-imidazo[1,2-a]pyridin-2-yl]-benzoesäure:
3-[8-(6-Aminohexanoyl-aminomethyl)-imidazo[1,2-a]pyridin-2-yl]-benzoesäure, F. 105 _{°} (Zers.);
aus 3-[8-(3-BOC-Aminopropionyl-aminomethyl)-imidazo[1,2-a]pyridin-2-yl]-benzoesäure:
3-[8-(3-Aminopropionyl-aminomethyl)-imidazo[1,2-a]pyridin-2-yl]-benzoesäure, F. 118° (Zers.);
aus 4-[8-(6-BOC-Aminohexanoyi-aminomethyl)-5,6,7,8-tetrahydro-imidazo[1,2-a]pyridin-2-yl]-benzoesäue:
4-[8-(6-Aminohexanoyl-aminomethyl)-5,6,7,8-tetrahydro-imidazo[1,2-a]pyridin-2-yl]-benzoesäure;
aus 4-[8-(3-BOC-Aminopropionyi-aminomethyl)-5,6,7,8-tetrahydro-imidazo[1,2-a]pyridin-2-yl]-benzoesäure:
4-[8-(3-Aminopropionyl-aminomethyl)-5,6,7,8-tetrahydro-imidazo[1,2-a]pyridin-2-yl]-benzoesäure;
aus 4-[8-(4-BOC-Aminomethylbenzoyl-aminomethyl)-5,6,7,8-tetrahydro-imidazo[1,2-a]pyridin-2-yl]-benzoesäure:
4-[8-(4-Aminomethylbenzoyl-aminomethyl)-5,6,7,8-tetrahydro-imidazo[1,2-a]pyridin-2-yl]-benzoesäure, F.
150 °(Zers.) (M + 1: 405);
aus 4-[8-(4-BOC-Amidinobenzoyl-aminomethyl)-imidazo[1,2-a]pyridin-2-yl]-benzoesäureethylester:
4-[8-(4-Amidinobenzoyl-aminomethyl)-imidazo[1,2-a]pyridin-2-yl]-benzoesäureethylester, F. 233-235 °;
aus 3-[3-(8-(3-BOC-Aminopropionyl-aminomethyl)-imidazo[1,2-a]pyridin-2-yl)-benzamido]-propionsäure:
3-[3-(8-(3-Aminopropionyl-aminomethyl)-imidazo[1,2-a]pyridin-2-yl)-benzamido]-propionsäure, amorph (M+1: 410);

### Beispiel 18

Analog Beispiel 12 erhält man ausgehend von 4-(8-BOC-Aminomethyl-5,6,7,8-tetrahydroimidazo[1,2-a]-pyridin-2-yl)-benzoesäure [erhältlich in Analogie zu Bsp. 7] durch Umsetzung mit β-Alanin-tert.-butylester in Gegenwart von 5,25 g N-(3-Dimethyl-aminopropyl)-N'-ethylcarbodiimid und 6 ml N-Methylmorpholin bei Raumtemperatur nach üblicher Aufarbeitung:
3-[4-(8-BOC-Aminomethyl-5,6,7,8-tetrahydroimidazo[1,2-a]pyridin-2-yl)-benzamido]-propionsäure-tert.-butylester.

Analog erhält man durch Umsetzung mit β-Alanin-tert.-butylester:
von 4-(7-BOC-Aminomethyl-5,6,7,8-tetrahydroimidazo[1,2-a]pyridin-2-yl)-benzoesäure:
3-[4-(7-BOC-Aminomethyl-5,6,7,8-tetrahydroimidazo[1,2-a]pyridin-2-yl)-benzamido]-propionsäure-tert.-butylester;
von 4-(7-BOC-Aminomethyl-imidazo[1,2-a]pyridin-2-yl)-benzoesäure:
3-[4-(7-BOC-Aminomethyl-imidazo[1,2-a]pyridin-2-yl)-benzamido]-propionsäure-tert.-butylester;
von 4-(6-BOC-Aminomethyl-5,6,7,8-tetrahydroimidazo[1,2-a]pyridin-2-yl)-benzoesäure:
3-[4-(6-BOC-Aminomethyl-5,6,7,8-tetrahydroimidazo[1,2-a]pyridin-2-yl)-benzamido]-propionsäuretert.- butylester;
von 4-(6-BOC-Aminomethyl-imidazo[1,2-a]pyridln-2-yl)-benzoesäure:
3-[4-(6-BOC-Aminomethyl-imidazo[1,2-a]pyridin-2-yl)-benzamido]-propionsäure-tert.-butylester;
von 3-(7-BOC-Aminomethyl-5,6,7,8-tetrahydroimidazo[1,2-a]pyridin-2-yl)-benzoesäure:
3-[3-(7-BOC-Aminomethyl-5,6,7,8-tetrahydroimidazo[1,2-a]pyridin-2-yl)-benzamido]-propionsäure-tert.-butylester;
von 3-(7-BOC-Aminomethyl-imidazo[1,2-a]pyridin-2-yl)-benzoesäure:
3-[3-(7-BOC-Aminomethyl-imidazo[1,2-a]pyridin-2-yl)-benzamido]-propionsäure-tert.-butylester;
von 3-(6-BOC-Aminomethyl-5,6,7,8-tetrahydroimidazo[1,2-a]pyridin-2-yl)-benzoesäure:
3-[3-(6-BOC-Aminomethyl-5,6,7,8-tetrahydroimidazo[1,2-a]pyridin-2-yl)-benzamido]-propionsäure-tert.-butylester;
von 3-(6-BOC-Aminomethyl-imidazo[1,2-a]pyridin-2-yl)-benzoesäure:
3-[3-(6-BOC-Aminomethyl-imidazo[1,2-a]pyridin-2-yl)-benzamido]-propionsäure-tert.-butylester.

### Beispiel 19

Analog Beispiel 8 erhält man durch Entfernung der Schutzgruppen mit Trifluoressigsäure aus den in Beispiel 18 hergestellten Verbindungen die folgenden Verbindungen in Form ihrer Trifluoracetate:
3-[4-(8-Aminomethyl-5,6,7,8-tetrahydroimidazo[1,2-a]pyridin-2-yl)-benzamido]-propionsäure, F. > 87 °(Zers.) (M + ₁: 343);
3-[4-(7-Aminomethyl-5,6,7,8-tetrahydroimidazo[1,2-a]pyridin-2-yl)-benzamido]-propionsäure; 3-[4-(7-Aminomethyl-imidazo[1,2-a]pyridin-2-yl)-benzamido]-propionsäure;
3-[4-(6-Aminomethyl-5,6,7,8-tetrahydroimidazo[1,2-a]pyridin-2-yl)-benzamido]-propionsäure; 3-[4-(6-Aminomethyl-imidazo[1,2-a]pyndin-2-yl)-benzamido]-propionsäure;
3-[3-(7-Aminomethyl-5,6,7,8-tetrahydroimidazo[1,2-a]pyridin-2-yl)-benzamido]-propionsäure; 3-[3-(7-Aminomethyl-imidazo[1,2-a]pyridin-2-yl)-benzamido]-propionsäure;
3-[3-(6-Aminomethyl-5,6,7,8-tetrahydroimidazo[1,2-a]pyridin-2-yl)-benzamido]-propionsäure; 3-[3-(6-Aminomethyl-imidazo[1,2-a]pyridin-2-yl)-benzamido]-propionsäure.

### Beispiel 20

Man löst 17,25 g 2-Aminopyridin-4-carbonsäure und 28 g 4-Brom-acetylbenzonitril in 200 ml N-Methylpyrrolidin und erhitzt 7 Std. auf 160 °. Nach Abkühlen wird die Reaktionsmischung filtriert und in 2 I Eiswasser eingegossen. Der entstandene Niederschlag wird abgesaugt und nacheinander mit 100 ml Wasser, 50 ml Ethanol und 50 ml Ether gewaschen. Man erhält 2-(4-Cyanphenyl)-imidazo[1,2-a]pyridin-7-carbonsäure-hydrobromid, F. 310° (Zers.).

Analog erhält man durch Umsetzung
von 2-Aminopyridin-5-carbonsäure mit 4-Bromacetylbenzonitril:
2-(4-Cyanphenyl)-imidazo[1,2-a]pyridin-6-carbonsäure-hydrobromid, F. > 330 ° (M+1: 264); von 2-Aminopyridin-6-carbonsäure mit 4-Bromacetylbenzonitril:
2-(4-Cyanphenyl)-imidazo[1,2-a]pyridin-5-carbonsäure-hydrobromid; von 2-Aminopyridin-3-carbonsäure mit 4-Bromacetylbenzonitril:
2-(4-Cyanphenyl)-imidazo[1,2-a]pyridin-8-carbonsäure-hydrobromid; von 2-Aminopyridin-5-carbonsäuremethylester mit 4-Bromacetylbenzonitril:
2-(4-Cyanphenyl)-imidazo[1,2-a]pyridin-6-carbonsäuremethylester-hydrobromid.

### Beispiel 21

1,32 g 2-(4-Cyanphenyl)-imidazo[1,2-a]pyridin-7-carbonsäure sowie 1,1 g Hydroxylamin-hydrochlorid werden in 125 ml Ethanol in Gegenwart von 2,24 g Natriummethanolat 2 Std. gekocht. Anschließend wird die Reaktionsmischung filtriert und im Vakuum eingeengt. Man löst den Rückstand in Wasser, stellt die Lösung mit 2 n Salzsäure auf pH 3 ein und saugt den entstehenden Niederschlag ab. Nach Umkristallisieren aus Wasser/Eisessig erhält man 2-[4-Amino-(hydroxyimino)methylphenyl]-imidazo[1,2-a]pyridin-7-carbonsäure, F. > 300 _{°} (Zers.) (M + ₁: 297).

Analog erhält man durch Umsetzung von Hydroxylamin-hydrochlorid
mit 2-(4-Cyanphenyl)-imidazo[1,2-a]pyridin-6-carbonsäure: 2-[4-Amino(hydroxyimino)methylphenyl]-imidazo[1,2-a]pyridin-6-carbonsäure;
mit 2-(4-Cyanphenyl)-imidazo[1,2-a]pyridin-5-carbonsäure: 2-[4-Amino(hydroxyimino)methylphenyl]-imidazo[1,2-a]pyridin-5-carbonsäure;
mit 2-(4-Cyanphenyl)-imidazo[1,2-a]pyridin-6-carbonsäuremethylester: 2-[4-Amino(hydroxyimino)methylphenyl]-imidazo[1,2-a]pyridin-6-carbonsäuremethylester;
mit 2-(4-Cyanphenyl)-imidazo[1,2-a]pyridin-8-carbonsäure: 2-[4-Amino(hydroxyimino)methylphenyl]-imidazo[1,2-a]pyridin-8-carbonsäure.

### Beispiel 22

5,2 g 2-(4-Cyanphenyl)-imidazo[1,2-a]pyridin-7-carbonsäure werden in 60 ml Dichlormethan und 15 ml DMF gelöst und nach Zugabe von 2,8 g 4-Piperidincarbonsäure-tert.-butylester in Gegenwart von 3,2 g N-(3-Dimethylaminopropyl)-N'-ethylcarbodiimid und 3,6 ml N-Methylmorpholin 24 Std. bei Raumtemperatur gerührt. Nach üblicher Aufarbeitung erhält man nach Verreiben mit Ether 1-[2-(4-Cyanphenyl)-imidazo[1,2-a]pyridin-7-yl-carbonyl]-piperidin-4-carbonsäure-tert.-butylester, F. 209 _{°} (Zers.).

Analog erhält man:
aus 2-(4-Cyanphenyl)-imidazo[1,2-a]pyridin-7-carbonsäure mit Gly-tert.-butylester:
2-[2-(4-Cyanphenyl)-imidazo[1,2-a]pyridin-7-yl-carboxamido]-essigsäure-tert.-butylester, F. 214°;
aus 2-(4-Cyanphenyl)-imidazo[1,2-a]pyridin-7-carbonsäure mit β-Ala-tert.-butylester:
3-[2-(4-Cyanphenyl)-imidazo[1,2-a]pyridin-7-yl-carboxamido]-propionsäure-tert.-butylester, F. 138-140°; aus 2-(4-Cyanphenyl)-imidazo[1,2-a]pyridin-6-carbonsäure mit 4-Piperidin-carbonsäure-tert.-butylester:
1-[2-(4-Cyanphenyl)-imidazo[1,2-a]pyridin-6-yl-carbonyl]-piperidin-4-carbonsäure-tert.-butylester, F. 183°;
aus 2-(4-Cyanphenyl)-imidazo[1,2-a]pyridin-6-carbonsäure mit Gly-tert.-butylester:
2-[2-(4-Cyanphenyl)-imidazo[1,2-a]pyridin-6-yl-carboxamido]-essigsäure-tert.-butylester, F. 321 _{°} (Zers.); aus 2-(4-Cyanphenyl)-imidazo[1,2-a]pyridin-6-carbonsäure mit β-Ala-tert.-butylester:
3-[2-(4-Cyanphenyl)-imidazo[1,2-a]pyridin-6-yl-carboxamido]-propionsäure-tert.-butylester, F. 187-190°.

### Beispiel 23

3,4 g 1-[2-(4-Cyanphenyl)-imidazo[1,2-a]pyridin-7-yl-carbonyl]-piperidin-4-carbonsäure-tert.-butylester werden in einer Mischung, enthaltend 44 ml Pyridin, 5,4 ml Triethylamin und 6 ml DMF, unter Eiskühlung 1,5 Std. mit H₂S-Gas behandelt. Anschließend wird 21 Std bei Raumtemperatur gerührt. Die Reaktionsmischung wird im Vakuum eingeengt und wie üblich aufgearbeitet. Man erhält 1-[2-(4-Thiocarbamoylphenyl)-imidazo[1,2-a]pyridin-7-yl-carbonyl]-piperidin-4-carbonsäure-tert.-butylester, F. 170 _{°} (Zers.).

Analog erhält man:
aus 2-[2-(4-Cyanphenyl)-imidazo[1,2-a]pyridin-7-yl-carboxamido]-essigsäure-tert.-butylester: 2-[2-(4-Thiocarbamoylphenyl)-imidazo[1,2-a]pyridin-7-yl-carboxamido]-essigsäure-tert.-butylester, F. 178°;
aus 3-[2-(4-Cyanphenyl)-imidazo[1,2-a]pyridin-7-yl-carboxamido]-propionsäure-tert.-butylester: 3-[2-(4-Thiocarbamoylphenyl)-imidazo[1,2-a]pyridin-7-yl-carboxamido]-propionsäure-tert.-butylester, F. 180°;
aus 1-[2-(4-Cyanphenyl)-imidazo[1,2-a]pyridin-6-yl-carbonyl]-piperidin-4-carbonsäure-tert.-butylester: 1-[2-(4-Thiocarbamoylphenyl)-imidazo[1,2-a]pyridin-6-yl-carbonyl]-piperidin-4-carbonsäure-tert.-butylester, F. 186°;
aus 2-[2-(4-Cyanphenyl)-imidazo[1,2-a]pyridin-6-yl-carboxamido]-essigsäure-tert.-butylester: 2-[2-(4-Thiocarbamoylphenyl)-imidazo[1,2-a]pyridin-6-yl-carboxamido]-essigsäure-tert.-butylester, F. 278 ° (Zers.);
aus 3-[2-(4-Cyanphenyl)-imidazo[1,2-a]pyridin-6-yl-carboxamido]-propionsäure-tert.-butylester: 3-[2-(4-Thiocarbamoylphenyl)-imidazo[1,2-a]pyridin-6-yl-carboxamido]-propionsäure-tert.-butylester, F. 296°.

### Beispiel 24

Man suspendiert 3,3 g 1-[2-(4-Thiocarbamoylphenyl)-imidazo[1,2-a]pyridin-7-yl-carbonyl]-piperidin-4-carbonsäure-tert.-butylester in 140 ml Aceton, fügt 19,2 ml Methyliodid hinzu und rührt 72 Std. bei Raumtemperatur. Der entstandene Niederschlag wird anschließend abgesaugt, mit 30 ml Aceton gewaschen und im Vakuum getrocknet. Man erhält 1-[2-(4-Methylsulfimidoylphenyl)-imidazo[1,2-a]pyridin-7-yl- carbonyl]-piperidin-4-carbonsäure-tert.-butylester-hydroiodid, F. 203 _{°} (Zers.).

Analog erhält man:
aus 2-[2-(4-Thiocarbamoylphenyl)-imidazo[1,2-a]pyridin-7-yl-carboxamido]-essigsäure-tert.-butylester: 2-[2-(4-Methylsulfimidoylphenyl)-imidazo[1,2-a]pyridin-7-yl-carboxamido]-essigsäure-tert.-butylester- hydroiodid, F. 158° (Zers.);
aus 3-[2-(4-Thiocarbamoylphenyl)-imidazo[1,2-a]pyridin-7-yl-carboxamido]-propionsäure-tert.-butylester: 3-[2-(4-Methylsulfmidoylphenyl)-imidazo[1,2-a]pyridin-7-yl-carboxamido]-propionsäure-tert.-butylester- hydroiodid, F. 169° (Zers.);
aus 1-[2-(4-Thiocarbamoylphenyl)-imidazo[1,2-a]pyridin-6-yl-carbonyl]-piperidin-4-carbonsäure-tert.-butylester:
1-[2-(4-Methylsulfimidoylphenyl)-imidazo[1,2-a]pyridin-6-yl-carbonyl]-piperidin-4-carbonsäure-tert.-butylester-hydroiodid, F. 205 ° (Zers.);
aus 2-[2-(4-Thiocarbamoylphenyl)-imidazo[1,2-a]pyridin-6-yl-carboxamido]-essigsäure-tert.-butylester:
2-[2-(4-Methylsulfimidoylphenyl)-imidazo[1,2-a]pyridin-6-yl-carboxamido]-essigsäure-tert.-butylester- hydroiodid, F. 185° (Zers.);
aus 3-[2-(4-Thiocarbamoylphenyl)imidazo[1,2-a]pyridin-6-yl-carboxamido]-propionsäuretert.-butylester:
3-[2-(4-Methylsulfimidoylphenyl)-imidazo[1,2-a]pyridin-6-yl-carboxamido]-propionsäure-tert.-butylester- hydroiodid, F. 178° (Zers.).

### Beispiel 25

Man löst 5,1 g 1-[2-(4-Methylsulfimidoylphenyl)-imidazo[1,2-a]pyridin-7-yl-carbonyl]-piperidin-4-carbonsäure-tert.-butylester in 400 ml Methanol, fügt 3,3 g Ammoniumacetat hinzu und rührt 21 Std. bei Raumtemperatur. Nach üblicher Aufarbeitung erhält man nach Verreiben mit Ether 1-[2-(4-Amidinophenyl)-imidazo[1,2-a]pyridin-7-yl-carbonyl]-piperidin-4-carbonsäuretert.-butylester, F. 221 °.

Analog erhält man:
aus 2-[2-(4-Methylsulfimidoylphenyl)-imidazo[1,2-a]pyridin-7-yl-carboxamido]-essigsäure-tert.-butylester:
2-[2-(4-Amidinophenyl)-imidazo[1,2-a]pyridin-7-yl-carboxamido]-essigsäure-tert.-butylester, F. 243°;
aus 3-[2-(4-Methylsulfimidoylphenyl)-imidazo[1,2-a]pyridin-7-yl-carboxamido]-propionsäure-tert.-butylester:
3-[2-(4-Amidinophenyl)imidazo[1,2-a]pyridin-7-yl-carboxamido]-propionsäure-tert.-butylester, F. 220°; aus 1-[2-(4-Methylsulfimidoylphenyl)-imidazo[1,2-a]pyridin-6-yl-carbonyl]-piperidin-4-carbonsäure-tert.-butylester:
1-[2-(4-Amidinophenyl)-imidazo[1,2-a]pyridin-6-yl-carbonyl]-piperidin-4-carbonsäure-tert.-butylester, F. 189 ° (Zers.);
aus 2-[2-(4-Methylsulfimidoylphenyl)-imidazo[1,2-a]pyridin-6-yl-carboxamido]-essigsäure-tert.-butylester:
2-[2-(4-Amidinophenyl)-imidazo[1,2-a]pyridin-6-yl-carboxamido]-essigsäure-tert.-butylester-hydroiodid, F. 281 _{°} (Zers.);
aus 3-[2-(4-Methylsulfimidoylphenyl)-imidazo[1,2-a]pyridin-6-yl-carboxamido]-propionsaure-tert.-butylester:
3-[2-(4-Amidinophenyl)-imidazo[1,2-a]pyridin-6-yl-carboxamido]-propionsäure-tert.-butylester-hydroiodid, F. 172 ° (Zers.).

### Beispiel 26

0,5 g 1-[2-(4-Amidinophenyl)-imidazo[1,2-a]pyridin-7-yl-carbonyl]-piperidin-4-carbonsäure-tert.-butylester werden in 25 ml mit HCI-Gas gesättigtem Essigsäureethylester 5 Std. bei Raumtemperatur gerührt. Der Niederschlag wird abgesaugt, mit 5 ml Essigsäureethylester gewaschen und anschließend in 30 ml Wasser suspendiert. Der pH-Wert der Suspension wird auf 10 eingestellt und 1 Std. im Eisbad nachgerührt. Nach Absaugen des Niederschlages, Waschen mit 10 ml Wasser und Vakuumtrocknung erhält man 1-[2-(4-Amidinophenyl)-imdazo[1,2-a]pyridin-7-yl-carbonyl]-piperidin-4-carbonsäure-Na-salz-Hydrat, F. 275 _{°} (Zers.).

Analog erhält man:
aus 3-[2-(4-Amidinophenyl)-imidazo[1,2-a]pyridin-7-yl-carboxamido]-propionsäure-tert.-butylester:
3-[2-(4-Amidinophenyl)-imidazo[1,2-a]pyridin-7-yl-carboxamido]-propionsäure-Bishydrochlorid-Trihydrat, F. 283 _{°} (Zers.).

### Beispiel 27

Analog Beispiel 1 erhält man durch Behandlung mit Trifluoressigsäure:
aus 2-[2-(4-Amidinophenyl)-imidazo[1,2-a]pyridin-7-yl-carboxamido]-essigsäure-tert.-butylester:
2-[2-(4-Amidinophenyl)-imidazo[1,2-a)pyridin-7-yl-carboxamido]-essigsäure-Bistrifluoracetat-Sesquihydrat, F. 255 ° (Zers.);
aus 1-[2-(4-Amidinophenyl)-imidazo[1,2-a]pyridin-6-yl-carbonyl]-piperidin-4-carbonsäure-tert.-butylester:
1-[2-(4-Amidinophenyl)-imidazo[1,2-a]pyridin-6-carbonyl]-piperidin-4-carbonsäure-Bistrifluoracetat, F. 230 ° (Zers.);
aus 2-[2-(4-Amidinophenyl)-imidazo[1,2-a]pyridin-6-yl-carboxamido]-essigsäure-tert.-butylester:
2-[2-(4-Amidinophenyl)-imidazo[1,2-a]pyridin-6-yl-carboxamido]-essigsäure-Bistrifluoracetat, F. 256°-(Zers.);
aus 3-[2-(4-Amidinophenyl)-imidazo[1,2-a]pyridin-6-yl-carboxamido]-propionsäure-tert.-butylester:
3-[2-(4-Amidinophenyl)-imidazo[1,2-a]pyridin-6-yl-carboxamido]-propionsäure-Bistrifluoracetat, F. 255°-(Zers.).

### Beispiel 28

1,6 g 3-(2-Aminopyridin-3-yl-carboxamido)-propionsäure-tert.-butylester [erhältlich durch Umsetzung von 2-Aminopyridin-3-carbonsäure mit β-Ala-tert.-butylester] und 1,1 g 4-Bromacetyl-benzonitril werden in 25 ml Ethanol gekocht. Nach beendeter Reaktion (DC-Kontrolle) wird der Niederschlag abfiltriert, mit Ethanol gewaschen und getrocknet. Man erhält 3-[2-(4-Cyanphenyl)-imidazo [1,2-a]pyridin-8-yl-carboxami- do]-propionsäure-tert.-butylester-Hydrobromid, F. 208-211 _{°} (M + 1: 391).

Analog erhält man durch Umsetzung von 4-Bromacetyl-benzonitril
mit 3-(2-Aminopyridin-4-yl-carboxamido)-propionsäure-tert.-butylester: 3-[2-(4-Cyanphenyl)-imidazo[1,2-a]pyridin-7-yl-carboxamido]-propionsäure-tert.-butylester;
mit 2-(2-Aminopyridin-4-yl-carboxamido)-essigsäure-tert.-butylester: 2-[2-(4-Cyanphenyl)-imidazo[1,2-a]pyridin-7-yl-carboxamido]-essigsäure-tert.-butylester;
mit 2-(2-Aminopyridin-4-yl-N-methylcarboxamido)-essigsäuretert.-butylester: 2-[2-(4-Cyanphenyl)-imidazo[1,2-a]pyridin-7-yl-N-methyl-carboxamido]-essigsäure-tert.-butylester;
mit 3-(2-Aminopyridin-4-yl-N-methylcarboxamido)-propionsäure-tert.-butylester: 3-[2-(4-Cyanphenyl)-imidazo[1,2-a]pyridin-7-yl-N-methyl-carboxamido]-propionsäure-tert.-butylester;
mit 2(S)-(2-Aminopyridin-4-yl-carboxamido)-bernsteinsäure-bis-tert.-butylester: 2(S)-[2-(4-Cyanphenyl)-imidazo[1,2-a]pyridin-7-yl-carboxamido]-bernsteinsäure-bis-tert.-butylester;
mit 2(R)-(2-Aminopyridin-4-yl-carboxamido)-bernstensäure-bis-tert.-butylester: 2(R)-[2-(4-Cyanphenyl)-imidazo[1,2-a]pyridin-7-yl-carboxamido]-bernsteinsäure-bis-tert.-butylester;
mit 1-(2-Aminopyridin-4-yl-carbonyl)-piperidin-4-carbonsäure-tert.-butylester: 1-[2-(4-Cyanphenyl)-imidazo[1,2-a]pyridin-7-yl-carbonyl]-piperidin-4-carbonsäure-tert.-butylester;
mit 1-(2-Aminopyridin-4-yl-carbonyl)-4-(tert.-butoxycarbonyl-methoxy)-piperidin:
1-[2-(4-Cyanphenyl)-imidazo[1,2-a]pyridin-7-yl-carbonyl]-4-(tert.-butyloxycarbonyl-methoxy)-piperidin; mit 1-(2-Aminopyridin-4-yl-carbonyl)-4-(tert.-butoxycarbonyl-methyl)-piperazin:
1-[2-(4-Cyanphenyl)-imidazo[1,2-a]pyridin-7-yl-carbonyl]-4-(tert.-butyloxycarbonyl-methyl)-piperazin; mit 1-(2-Aminopyndin-4-yl-carbonyl)-4-[2-(tert.-butoxycarbony)-ethy]-piperazin:
1-[2-(4-Cyanphenyl)-imidazo[1,2-a]pyridin-7-yl-carbonyl]-4-[2-(tert.-butoxycarbonyl)-ethyl]-piperazin.

### Beispiel 29

0,5 g 3-[2-(4-Cyanphenyl)-imidazo[1,2-a]pyridin-8-yl-carboxamido]-propionsäure-tert.-butylester werden in 10 ml einer THF/Dioxan-Mischung (1:1), unterZusatz von 2 ml DMF, in Gegenwart von 0,14 g KOH und 0,76 g K₂C0₃ 2 Std. bei Raumtemperatur gerührt. Nach Entfernung des Lösungsmittels wird der Rückstand in Wasser aufgenommen und filtriert. Nach üblicher Aufarbeitung erhält man 3-[2-(4-Cyanphenyl)-imidazo-[1,2-a]pyridin-8-yl-carboxamido]-propionsäure, F. 243-248 _{°} (Zers.) (M + 1: 335). Analog erhält man durch Verseifung der entsprechenden Ester aus Beispiel 28: 3-[2-(4-Cyanphenyl)-imidazo[1,2-a]pyridin-7-yl-carboxamido]-propionsäure; 2-[2-(4-Cyanphenyl)-imidazo[1,2-a]pyridin-7-yl-carboxamido]-essigsäure; 2-[2-(4-Cyanphenyl)-imidazo[1,2-a]pyridin-7-yl-N-methyl-carboxamido]-essigsäure; 3-[2-(4-Cyanphenyl)-imidazo[1,2-a]pyridin-7-yl-N-methyl-carboxamido]-propionsäure; 2(S)-[2-(4-Cyanphenyl)-imidazo[1,2-a]pyridin-7-yl-carboxamido]-bernsteinsäure; 2(R)-[2-(4-Cyanphenyl)-imidazo[1,2-a]pyridin-7-yl-carboxamido]-bernsteinsäure; 1-[2-(4-Cyanphenyl)-imidazo[1,2-a]pyridin-7-yl-carbonyl]-piperidin-4-carbonsäure; 1-[2-(4-Cyanphenyl)-imidazo[1,2-a]pyridin-7-yl-carbonyl]-4-(carboxymethoxy)-piperidin; 1-[2-(4-Cyanphenyl)-imidazo[1,2-a]pyridin-7-yl-carbonyl]-4-(carboxymethyl)-piperazin; 1-[2-(4-Cyanphenyl)-imidazo[1,2-a]pyridin-7-yl-carbonyl]-4-(2-carboxyethyl)-piperazin.

### Beispiel 30

0,27 g 3-[2-(4-Cyanphenyl)-imidazo[1,2-a]pyridin-8-yl-carboxamido]-propionsäure werden analog zu Beispiel 3 mit H₂S-Gas umgesetzt. Man erhält 3-[2-(4-Thiocarbamoylphenyl)-imidazo [1,2-a]pyridin-8-yI-carboxa- mido]-propionsäure, F 267-268 ° (Zers.) (M + 1: 369).

Analog erhält man durch Umsetzung der Verbindungen aus Beispiel 29 mit H₂S: 3-[2-(4-Thiocarbamoylphenyl)-imidazo[1,2-a]pyridin-7-yl-carboxamido]-propionsäure; 2-[2-4-Thiocarbamoylphenyl)-imidazo[1,2-a]pyridin-7-yl-carboxamido]-essigsäure; 2-[2-(4-Thiocarbamoylphenyl)-imidazo[1,2-a]pyridin-7-yl-N-methylcarboxamido]-essigsäure; 3-[2-(4-Thiocarbamoylphenyl)-imidazo[1,2-a]pyridin-7-yl-N-methylcarboxamido]-propionsäure; 2(S)-[2-(4-Thiocarbamoylphenyl)-imidazo[1,2-a]pyridin-7-yl-carboxamido]-bernsteinsäure; 2(R)-[2-(4-Thiocarbamoylphenyl)-imidazo[1,2-a]pyridin-7-yl-carboxamido]-bernsteinsäure; 1-[2-(4-Thiocarbamoylphenyl)-imidazo[1,2-a]pyridin-7-yl-carbonyl]-piperidin-4-carbonsäure; 1-[2-(4-Thiocarbamoylphenyl)-imidazo[1,2-a]pyridin-7-yl-carbonyl]-4-(carboxy-methoxy)-piperidin; 1-[2-(4-Thiocarbamoylphenyl)-imidazo[1,2-a]pyridin-7-yl-carbonyl]-4-(carboxy-methyl)-piperazin; 1-[2-(4-Thiocarbamoylphenyl)-imidazo[1,2-a]pyridin-7-yl-carbonyl]-4-(2-carboxy-ethyl)-piperazin.

### Beispiel 31

0,2 g 3-[2-(4-Thiocarbamoylphenyl)-imidazo[1,2-a]pyridin-8-yl-carboxamido]-propionsäure werden analog zu Beispiel 4 in Aceton mit Methyliodid umgesetzt. Man erhält 3-[2-(4-Methylsulfimidoylphenyl)-imidazo-[1,2-a]pyridin-8-yl-carboxamido]-propionsäure.

Analog erhält man durch Umsetzung der Verbindungen aus Beispiel 30 mit Methyliodid: 3-[2-(4-Methylsulfimidoylphenyl)-imidazo[1,2-a]pyridin-7-yl-carboxamido]-propionsäure; 2-[2-(4-Methylsulfmidoylphenyl)-imidazo[1,2-a]pyridin-7-yl-carboxamido]-essigsäure; 2-[2-(4-Methylsulfimidoylphenyl)-imidazo[1,2-a]pyridin-7-yl-N-methylcarboxamido]-essigsäure; 3-[2-(4-Methylsulfimidoylphenyl)-imidazo[1,2-a]pyridin-7-yl-N-methylcarboxamido]-propionsäure; 2(S)-[2-(4-Methylsulfimidoylphenyl)-imidazo[1,2-a]pyridin-7-yl-carboxamido]-bernsteinsäure; 2(R)-[2-(4-Methylsulfimidoylphenyl)-imidazo[1,2-a]pyridin-7-yl-carboxamido]-bernsteinsäure; 1-[2-(4-Methylsulfimidoylphenyl)-imidazo[1,2-a]pyridin-7-yl-carbonyl]-piperidin-4-carbonsäure; 1-[2-(4-Methylsulfimidoylphenyl)-imidazo[1,2-a]pyridin-7-yl-carbonyl]-4-(carboxy-methoxy)-piperidin; 1-[2-(4-Methylsulfimidoylphenyl)-imidazo[1,2-a]pyridin-7-yl-carbonyl]-4-(carboxy-methyl)-piperazin; 1-[2-(4-Methylsulfimidoylphenyl)-imidazo[1,2-a]pyridin-7-yl-carbonyl]-4-(2-carboxy-ethyl)-piperazin.

### Beispiel 32

4,0 g 4-(8-Cyanimidazo[1,2-a]pyridin-2-yl)-benzoesäure und 2 ,14 g 3-Aminopropionsäure-ethylester werden in 40 ml DMF in Gegenwart von 2,28 g Hydrorybenztriazol, 3,0 g N-Methylmorpholin und 2,9 g N-(3-Dimethylaminopropyl)-N'-ethylcarbodiimid bei Raumtemperatur 4 Std. gerührt. Nach Zugabe von 400 ml Wasser wird der Niederschlag abgesaugt und nacheinander mit verdünnter Bicarbonatlösung und Wasser gewaschen. Man erhält 3-[4-(8-Cyanimidazo[1,2-a]-pyridin-2-yl)-benzamido]-propionsäuremethylester, F. 201-204°.

### Beispiel 33

0,55 g 3-[4-(8-Cyanimidazo[1,2-a]pyridin-2-yl)-benzamido]-propionsäurebenzylester [erhältlich durch Umsetzung von 4-(8-Cyan-imidazo[1,2-a]pyridin-2-yl)-benzoesäure mit 3-Aminopropionsäure-benzylester gemäß Bsp. 32] werden in 30 ml Methanol mit 0,27 g Hydroxylamin-Hydrochlorid in Gegenwart von 0,72 g K₂C0₃ 4 Std. gekocht. Nach Abkühlen wird der Niederschlag abgesaugt und nacheinander mit Wasser und Methanol gewaschen. Man erhält 3-[4-(8-Cyanimidazo[1,2-a]pyridin-2-yl)-benzamido]-propionsäure, F. 212-215°.

### Beispiel 34

Analog Beispiel 25 erhält man aus den entsprechenden Methylsulfimidoyl-Derivaten aus Beispiel 31 die nachfolgenden Amidino-Derivate:
3-[2-(4-Amidinophenyl)-imidazo[1,2-a]pyridin-7-yl-carboxamido]-propionsäure; 2-[2-(4-Amidinophenyl)-imidazo[1,2-a]pyridin-7-yl-carboxamido]-essigsäure; 2-[2-(4-Amidinophenyl)-imidazo[1,2-a]pyridin-7-yl-N-ethyl-carboxamido]-essigsäure; 3-[2-(4-Amidinophenyl)-imidazo[1,2-a]pyridin-7-yl-N-methyl-carboxamido]-propionsäure; 2(S)-[2-(4-Amidinophenyl)-imidazo[1,2-a]pyridin-7-yl-carboxamido]-bernsteinsäure; 2(R)-[2-(4-Amidinophenyl)-imidazo[1,2-a]pyridin-7-yl-carboxamido]-bernsteinsäure; 1-[2-(4-Amidinophenyl)-imidazo[1,2-a]pyridin-7-yl-carbonyl]-piperidin-4-carbonsäure; 1-[2-(4-Amidinophenyl)-imidazo[1,2-a]pyridin-7-yl-carbonyl]-4-(carboxymethoxy)-piperidin; 1-[2-(4-Amidinophenyl)-imidazo[1,2-a]pyridin-7-yl-carbonyl]-4-(carboxymethyl)-piperazin; 1-[2-(4-Amidinophenyl)-imidazo[1,2-a]pyridin-7-yl-carbonyl]-4-(2-carboxyethyl)-piperazin.

Die nachstehenden Beispiele betreffen pharmazeutische Zubereitungen.

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### BeispielC: Lösung

Man bereitet eine Lösung aus 1 g eines Wirkstoffes der Formel I, 9,38 g NaH₂P0₄ x 2 H₂0, 28,48 g Na2 HP04 x 12 H₂0 und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen jede Ampulle enthält 10 mg Wirkstoff.

## Patentansprüche

1. Verbindungen der Formel I 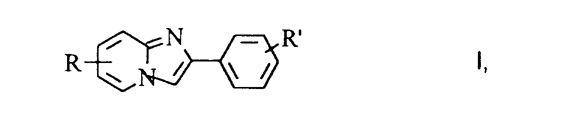
sowie die entsprechenden 5,6,7,8-Tetrahydro-imidazo[1,2-a]pyridin-Derivate,
worin
R Q oder COX,
R' Q, sofern R = COX und COX, sofern R = Q ist,
Q N0₂, NH₂, CN, CSNH₂, C(=NH)S-A, C(=NH)NHOH, C(=NH)-NH₂, CH₂-NH₂, CH₂NH-C(=NH)-NH₂, NH-C(=NH)-NH₂, CH₂NHCO-alk-NH₂, CH₂NHCO-Ar-C-(=NH)NH₂, CH₂ NHCO-Ar-CH₂-NH₂ oder D, D 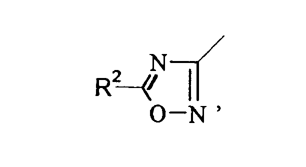
X OH, OA, AS, AS-AS', 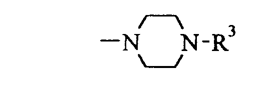 oder 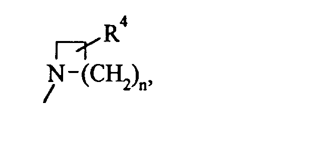
AS oder AS' jeweils unabhängig voneinander einen Aminosäurerest, ausgewählt aus einer Gruppe bestehend aus Ala, β-Ala, Arg, Asn, Asp, Gln, Glu, Gly, Leu, Lys, Orn, Phe, Pro, Sar, Ser, Thr, Tyr, Val, C-Allyl-Gly, C-Propargyl-Gly, N-Benzyl-Gly, N-Phenethyl-Gly, N-Benzyl-ß-Ala, N-Methyl-ß-Ala und N-Phenethyl-ß-Ala, wobei freie Amino-oder Carboxyl-Gruppen auch mit an sich bekannten Schutzgruppen versehen sein können,
_{R}2 OH, A oder Ar,
R³ -(CH₂)ₘ-COOR⁵,
R⁴ -(CH₂)p-COOR⁵ oder-(CH₂)_{q}-O-(CH₂)ᵣ₋COOR⁵
_{R}5 H oder A,
m 1, 2 oder 3,
n 1,2, 3 oder 4,
p 0, 1 oder 2,
q 0 oder 1,
r 1 oder 2,
A Alkyl mit 1 bis 6 C-Atomen,
-alk- Alkylen mit 1 bis 6 C-Atomen
und
Ar Phenylen
bedeuten,
sowie ihre physiologisch unbedenklichen Salze und/oder Solvate.

2. Enantiomere oder Diastereomere der Verbindungen der Formel I gemäß Anspruch 1.

3. Verbindungen der Formel I gemäß Anspruch 1, worin die freien Amino-, Amidino- oder Guanidinogruppen teilweise oder vollständig durch konventionelle Amino-Schutzgruppen geschützt sind. 4.
(a) 3-[4-(7-Amidinoimidazo[1,2-a]pyridin-2-yl)-benzamido]-propionsäure;
(b) 3-[4-(7-Amidinomethyl-imidazo[1,2-a]pyridin-2-yl)-benzamido]-propionsäure-tert.-butylester;
(c) 1-[4-(6-Aminomethyl-imidazo[1,2-a]pyridin-2-yl)-benzoyl]-piperidin-4-carbonsäure;
(d) 3-[4-(6-Amidino-imidazo[1,2-a]pyridin-2-yl)-benzamido]-propionsäure;
(e) 2-[2-(4-Amidinophenyl)-imidazo[1,2-a]pyridin-7-yl-carboxamido]-essigsäure;
(f) 3-[2-(4-Amidinophenyl)-imidazo[1,2-a]pyridin-7-yl-carboxamido]-propionsäure;
(g) 2-[2-(4-Amidinophenyl)-imidazo[1,2-a]pyridin-6-yl-carboxamido]-propionsäure
gemäß Anspruch 1, sowie deren Salze und/oder Solvate.

5. Verfahren zur Herstellung von Verbindungen der Formel I gemäß Anspruch 1, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Verbindung der Formel II 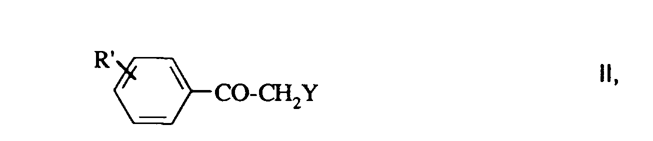
worin
R' die angegebene Bedeutung hat und
Y Cl, Br, I, OH oder eine reaktionsfähige veresterte OH-Gruppe bzw. leicht nucleophil substituierbare Abgangsgruppe bedeutet,
mit einem Ryridinderivat oder Formel 111 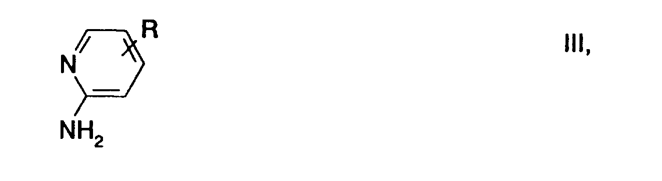
worin R die angegebene Bedeutung besitzt, umsetzt, oder daß man
c) einen Rest R und/oder R' in einen anderen Rest R und/oder R' umwandelt, indem man
- einen Ester der Formel I verseift, oder
- eine Carbonsäure der Formel I verestert, in ein Amid überführt oder unter den Bedingungen der Peptidsynthese mit einer Aminosäure oder einem Dipeptid verknüpft, oder
- eine N0₂- und/oder CN-Gruppe katalytisch hydriert, oder
- eine Nitrilgruppierung durch Anlagerung von Ammoniak in eine C(=NH)-NH₂-Gruppe umwandelt, oder
- eine Nitrilgruppe in eine Thiocarbamoylgruppe umwandelt, oder
- eine Thiocarbamoylgruppe in eine Methylsulfimidogruppe überführt oder
- eine Methylsulfimidogruppe in eine Amidin-Gruppe überführt
- eine Nitrilgruppe durch Anlagerung von NH₂0H in eine C(=NH)-NHOH-Gruppe umwandelt, oder
- eine C(= NH)-NHOH-Gruppe in eine Amidin-Gruppe umwandelt
- eine CH₂NH₂-Gruppe in eine Alkanoylaminomethylgruppe umwandelt, oder
- eine 1,2,4-Oxadiazol- bzw. 1,2,4-Oxadiazolinon-Gruppe in eine Amidin-Gruppe umwandelt, und/oder daß man
d) eine Verbindung der Formel I durch Behandeln mit einer Saure oder einer Base in eines ihrer Salze überführt.

6. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

7. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

8. Verwendung einer Verbindung der Formel I gemäß Anspruch 1 und/oder eines ihrer physiologisch unbedenklichen Salze bei der Bekämpfung von Krankheiten.

9. Verwendung einer Verbindung der Formel I gemäß Anspruch 1 und/oder eines ihrer physiologisch unbedenklichen Salze zur Herstellung von Arzneimitteln.
